# EUROPÄISCHE PATENTSCHRIFT

(11) **EP 3 949 310 B1**
(45) Veröffentlichungstag und Bekanntmachung des Hinweises auf die Patenterteilung: **10.09.2025**
(21) Anmeldenummer: 20707114.3
(22) Anmeldetag: 04.03.2020
(51) Int. Cl.: H04L 9/08, H04L 9/32, H04L 9/40, H04W 12/00, H04W 12/50, H04W 84/18, A61M 5/172, A61M 5/142

(54) **SICHERE KOMMUNIKATIONSVERBINDUNG ZWISCHEN MEDIZINISCHEN GERÄTEN EINER DATENMANAGEMENTVORRICHTUNG**
SECURE COMMUNICATION LINK BETWEEN MEDICAL DEVICES OF A DATA MANAGEMENT DEVICE
CONNEXION DE COMMUNICATION SÉCURISÉE ENTRE DES DISPOSITIFS MÉDICAUX D'UN DISPOSITIF DE GESTION DE DONNÉES

(30) Priorität: 28.03.2019 EP 19165723
(43) Veröffentlichungstag der Anmeldung: 09.02.2022
(60) Teilanmeldung: 25194346.0
(73) Patentinhaber: Ypsomed AG, 3401 Burgdorf (CH)
(72) Erfinder: LABUDDE, Marc, 3095 Spiegel (CH); LINDEGGER, Stefan, 4950 Huttwil (CH); LEUZINGER, Thomas, 3110 Münsingen (CH); ZENGER, Mathias, 3400 Burgdorf (CH); WYSS, Adrian, 3008 Bern (CH)
(74) Vertreter: Meier Obertüfer, Jürg
(86) Internationale Anmeldenummer: PCT/EP2020/055604
(87) Internationale Veröffentlichungsnummer: WO 2020/193090

(56) Entgegenhaltungen:
- EP-A1- 2 320 621
- EP-A1- 3 051 744
- US-A1- 2014 281 547
- ALEXIS DUQUE: "Deep Dive into Bluetooth LE Security - Rtone IoT Security - Medium", 3 March 2018 (2018-03-03), XP055600523, Retrieved from the Internet <URL:https://medium.com/rtone-iot-security/deep-dive-into-bluetooth-le-security-d2301d640bfc> [retrieved on 20190628]

## Beschreibung

### TECHNISCHES GEBIET

Die vorliegende Erfindung betrifft das Gebiet der Selbsttherapie mittels Injektions- und Infusionsgeräten sowie dem damit verbundenen Management von Therapiedaten, insbesondere dem Etablieren von sicheren Datenübertragungsverbindungen zwischen therapiebezogenen Geräten und Datenmanagementvorrichtungen.

### HINTERGRUND DER ERFINDUNG

Portable medizinische Geräte, wie Injektionsgeräte, portable Infusionspumpen oder auch Blutzuckermessvorrichtungen, verfügen heute über die Möglichkeit, Daten drahtlos auf andere Geräte zu übertragen. Beispielhaft können Blutzuckermesswerte von einer Blutzuckermessvorrichtung auf ein Diabetesmanagementgerät (insbesondere ein Mobiltelefon) via Bluetooth übertragen werden. Ergänzend können beispielhaft Verabreichungsgeräte wie Insulinpumpen die Verabreichungshistorie via Bluetooth an dasselbe Diabetesmanagementgerät übertragen. Umgekehrt ist es möglich Anweisungen vom Datenmanagementgerät an zum Beispiel ein Verabreichungsgerät zu Übertragen. Die übertragenen Daten sind einerseits im Falle von Therapiedaten oder Messdaten sensible Daten und zumindest im Falle von Therapieanweisungsdaten patientenkritische Daten, die vor Fremdzugriff gesichert werden müssen. Die Bluetoothtechnologie stellt standardmässig bereits verschiedene Möglichkeiten bereit, um drahtlose Verbindungen auf der Transportebene abhörsicher gestalten zu können. Die bereitgestellten Möglichkeiten decken jedoch nicht alle denkbaren Angriffsszenarien ab, weshalb es Sinn macht, auf Applikationsebene weitere Sicherheitsmassnahmen (insbesondere eine zusätzliche Verschlüsselung der Daten) umzusetzen.

Aus der WO2017200989 A1 sind Geräte und Verfahren bekannt, bei welchen in allen Geräten, die miteinander Daten austauschen sollen, ein und derselbe statische Schlüssel hinterlegt ist, um zusätzliche Sicherheit in der Applikationsebene zu etablieren. Das hat den Nachteil, dass auf allen Geräten des Systems derselbe Schlüssel gespeichert ist. US20140281547 Al offenbart out-of-band Übertragung von Pairing-Informationen zwischeneinem persönlichen medizinischen Gerät und einem Computer.Die Literaturstelle im Internet: Alexis Duque, ..Deep Dive into Bluetooth LE Security" auf med i um. com, https://medium.com/ rtone-iot-security/deep-dive-into-bluetooth-le-sccurity d2301d640bfc (abgerufen am 01.03.2022), zitiert beim EPA als Dokument XP055600523, offenbart verschiedene Bluetooth Pairing Methoden, darunter Bluetooth just-works.

In einem alternativen Ansatz zur Verbesserung der Sicherheit einer Datenaustauschverbindung zwischen Glukosemonitor (oder Patch Pumpe) und einer Kontrolleinheit ist aus der WO2016092448 A1 die Möglichkeit bekannt, dass zur Bildung eines Schlüssels für die Bluetoothverbindung ein im Glukosemonitor (oder in der Patch Pumpe) hinterlegtes Einmalpasswort verwendet werden kann. Das heisst, nach einmaligem Gebrauch wird das Passwort blockiert, so dass keine Verbindung mit einem anderen Gerät eingegangen werden kann. Nachteilig ist an dieser Lösung, dass ein Glukosemonitor (oder eine Patch Pumpe) immer an derselben Kontrolleinheit verwendet werden muss.

### DARSTELLUNG DER ERFINDUNG

Es ist eine Aufgabe der Erfindung, alternative Verfahren und Systeme für das Etablieren von sicheren Datenkommunikationsverbindungen zwischen portablen medizinischen Geräten und Datenmanagementvorrichtungen bereitzustellen.

Die Aufgabe wird gelöst durch Verfahren und System gemäss den unabhängigen Ansprüchen. Vorteilhafte Weiterbildungen und Ausführungen sind in den abhängigen Ansprüchen sowie der Beschreibung und den Zeichnungen dargelegt.

Ein Aspekt der Erfindung ist ein Verfahren zum Etablieren einer sicheren Datenkommunikationsverbindung zwischen einem portablen medizinischen Gerät (MG), insbesondere einem Infusionsgerät, einem Injektionsgerät oder einer Blutzuckermessvorrichtung, und einer Datenmanagementvorrichtung (DV). Der Begriff "Daten" ist dabei breit auszulegen und kann im Kontext des vorliegenden Dokuments Informationen, wie zum Beispiel historische Daten, Zustandsinformation oder Einstellungen des MG, umfassen. Weiter kann der Begriff aber auch Einstellungen, Befehle und Anweisungen, welche von Gerät zu Gerät übertragen werden, umfassen. Auch kann der Begriff "Daten" Authentifizierungsinformationen oder Signaturen im Rahmen der Kommunikation mit meinen. Der Begriff "medizinisches Gerät" umfasst (intelligente) Geräte, welche von einem Patienten selbst bedient werden können und bevorzugt am Patienten getragen werden können, insbesondere zur subkutanen Verabreichung von Medikamenten oder zur Messung von physiologischen Messgrössen, und welche mit den notwendigen (elektronischen) Prozessoreinheiten ausgestattet sind. Die Geräte umfassen auch (intelligente) Zusatzgeräte, welche lösbar mit einem mechanischen Injektionsgerät verbunden werden können.

Das erfindungsgemässe Verfahren umfasst in einem Aspekt zumindest die folgenden Schritte. Vom MG wird out-of-band (OOB) ein öffentlicher Schlüssel sowie optional weitere Informationen auf die DV übertragen. Weiter bauen MG und DV unabhängig vom übertragenen öffentlichen Schlüssel eine Bluetooth (BT) Verbindung auf, welche verschlüsselt ist. Nach Aufbau dieser BT-Verbindung übermittelt die DV einen öffentlichen Schlüssel zum MG über die aufgebaute BT-Verbindung. Aus dem von MG auf DV übertragenen öffentlichen Schlüssel und einem geheimen Schlüssel der DV berechnet die DV ein gemeinsames Geheimnis und umgekehrt berechnet das MG aus dem von DV auf MG übertragenen öffentlichen Schlüssel und einem geheimen Schlüssel des MG dasselbe gemeinsame Geheimnis wie die DV. In der Folge wird auf Basis des gemeinsamen Geheimnisses unter Ableitung eines gemeinsamen Schlüssels (der mit dem gemeinsamen Geheimnis übereinstimmen kann aber nicht muss), welcher durch MG und DV berechnet wurde, eine verschlüsselte End-zu-End-Verbindung aufgebaut. Diese End-zu-End-verschlüsselte Verbindung wird im sogenannten application security layer (ASL) erstellt, also auf Anwendungsebene, während die BT-Verbindung auf der Transportebene ebenfalls eine Verschlüsselung umfasst.

Gegenüber dem Stand der Technik hat die Erfindung den Vorteil, dass einerseits der standardisierten Verschlüsselung durch das BT-Protokoll eine zusätzliche Verschlüsselung hinzugefügt wird und andererseits ein MG durchaus (hintereinander) mit unterschiedlichen DVs verbunden werden kann.

In einem Aspekt der Erfindung wird der öffentliche Schlüssel vom MG auf die DV mittels Nahfeldkommunikation, insbesondere NFC, übertragen. Nahfeldkommunikation hat den Vorteil, dass sie zwischen Geräten nur dann funktioniert, wenn die Geräte (geometrisch) sehr nahe beieinander sind, im Bereich von Zentimetern, was das Abhören der Kommunikation erschwert.

In einem Aspekt der Erfindung zeigt das MG den zu übertragenen öffentlichen Schlüssel auf zum Beispiel einem Display an. In möglichen Ausgestaltungen kann der Schlüssel hierbei bevorzugt dynamisch generiert werden und beispielhaft für einen bestimmten Zeitraum auf dem bespielhaft erwähnten Display angezeigt werden. Die DV kann zum Empfang des öffentlichen Schlüssels vom MG ein passendes optisches Mittel, insbesondere eine Kamera, umfassen, mit welchem der angezeigte Schlüssel aufgezeichnet wird. Der Schlüssel kann vom MG als Barcode, QR-Code, durch eine andere graphische Repräsentation oder auch textuell dargestellt werden.

In einem alternativen Aspekt der Erfindung kann der öffentliche Schlüssel, welcher vom MG auf die DV übertragen wird, am MG angeordnet sein. Hierzu kann der Schlüssel auf dem Gehäuse des MG aufgedruckt oder (z. B. in Form einer Etikette) aufgeklebt sein, so dass er über eine Kamera an der DV in der DV empfangen werden kann. In einer weiteren Ausgestaltung, kann der der Schlüssel an der Verpackung des MG oder auf einer Beilage der Verpackung angeordnet sein. Der Schlüssel kann hierbei als Barcode, QR-Code, eine andere graphische Repräsentation oder auch textuell dargestellt sein.

In einem Aspekt der Erfindung erfolgt der Aufbau der BT-Verbindung, insbesondere Bluetooth LE, nach dem just-works-Prinzip, wie es in der Bluetooth-Spezifikation (https://www.bluetooth.com/specifications/archived-specifications; Version 5: Bluetooth Core Specification V 5.0, Dec 06, 2016) beschrieben ist, welche hiermit durch Verweis vollständig in das vorliegende Dokument aufgenommen ist. Bevorzugt findet beim Etablieren der BT Verbindung ein Diffie-Hellman-artiger Schlüsseltausch statt, wie nach der BT-Spezifikation möglich. In einer möglichen Ausgestaltung werden die Schlüssel mittels Elliptic Curve Diffie-Hellman P256 Methode generiert. Hierbei tauschen das MG und die DV auf der Bluetooth-Transportebene via die BT-Verbindung (welche dann noch unverschlüsselt ist) öffentliche Schlüssel aus und generieren parallel zueinander aus dem jeweils empfangenen öffentlichen Schlüssel des anderen Gerätes und einem eigenen geheimen Schlüssel ein gemeinsames Geheimnis. Dieses gemeinsame Geheimnis ist die Basis für einen gemeinsamen Langzeitschlüssel, welcher für die weitere Kommunikation zwischen den beiden Geräten als Basis dient. Das gemeinsame Geheimnis kann dabei den Langzeitschlüssel darstellen, oder er wird aus dem gemeinsamen Geheimnis ein Langzeitschlüssel einmalig, wiederholt oder periodisch abgeleitet werden. Der Langzeitschlüssel ist dabei erfindungsgemäss 128 Bit lang oder länger. Zwischen MG und DV ist somit eine verschlüsselte BT-Verbindung aufgebaut, über welche die DV ihren öffentlichen Schlüssel übertragen kann, und über welche die erfindungsgemäss End-zu-End-verschlüsselten Daten noch einmal verschlüsselt werden.

In einem Aspekt der Erfindung werden für die End-zu-End-Verschlüsselung (analog) ebenfalls Langzeitschlüssel generiert, welche validiert und nach erfolgreicher Validierung in beiden Geräten auf der Applikationsebene gespeichert werden.

In einem Aspekt der Erfindung wird das Paar aus öffentlichem und geheimem Schlüssel für die End-zu-End-Verschlüsselung im MG und/oder der DV dynamisch generiert. Zum Beispiel kann ein Schlüsselpaar für jede neue Verbindung von einem MG zu einer DV im MG und/oder in der DV neu generiert werden. Alternativ ist es auch möglich, dass in einem MG ein Schlüsselpaar beim ersten Start des MG generiert wird, und das Schlüsselpaar danach nicht mehr verändert wird. In einer weiteren Alternative wird das Schlüsselpaar des MG noch im Produktionswerk, insbesondere im Rahmen der Gerätetestung generiert und danach nicht mehr verändert. In einer weiteren erfindungsgemässen Ausgestaltung wird das Schlüsselpaar für die End-zu-End-Verschlüsselung in der DV bei der Installation der Software auf der Vorrichtung generiert. Alternativ wird zum Beispiel bei jeder neuen Verbindung ein neues Schlüsselpaar generiert oder regelmässig (zeitabhängig) erneuert.

In einem Aspekt der Erfindung betrifft die Erfindung ein System, welches aus mindestens einem MG und einer DV besteht, zwischen welchen eine sichere und erfindungsgemässe Datenübertragungsverbindung etablierbar ist. In einer Ausgestaltung dieses Aspektes ist die DV ein Mobiltelefon, insbesondere ein Smart Phone (wie ein Apple iPhone) oder ein Handheld Computer (wie ein Tablet, beispielhaft in Form eines Apple iPads. In einer weiteren alternativen Ausgestaltung kann die DV auch als dediziertes Datenmanagement und Steuergerät für das mindestens eine MG ausgestaltet sein, insbesondere als Personal Diabetes Manager (PDM). In einer weiteren Alternative kann die DV auch ein PC oder PC Notebook sein. In einer Ausgestaltung dieses Aspektes kann das MG als Infusionspumpe insbesondere als Insulinpumpe ausgestaltet sein. In einer Variante dieser Ausgestaltung kann es sich um eine konventionelle Insulinpumpe handeln. In einer weiteren Variante dieser Ausgestaltung kann es sich bei der Insulinpumpe um eine sogenannte Patch Pumpe handeln, welche direkt auf der Haut getragen wird. Die Patch Pumpe kann hierbei insbesondere auch modular aufgebaut sein und zumindest aus einem Einwegmodul, welches ein Reservoir umfasst, sowie einem Mehrwegmodul bestehen, welches zumindest Teile der Steuerelektronik umfasst. Die konventionelle Insulinpumpe umfasst dabei eine Anzeige, auf welcher der öffentliche Schlüssel der Insulinpumpe für die End-zu-Endverschlüsselung erfindungsgemässe für die OOB-Übertragung darstellbar ist. Alternativ kann der öffentliche Schlüssel auch auf dem Gehäuse fest angeordnet sein. Die Patch Pumpe kann zur OOB-Übertragung ein NFC-Tag umfassen, auf welchem der insbesondere dynamisch generierte öffentliche Schlüssel lesbar abgelegt ist. Alternativ kann auch bei der Patch Pumpe der öffentliche Schlüssel auf das Gehäuse gedruckt sein. Auch bei der konventionellen Insulinpumpe kann die OOB-Übertragung des öffentlichen Schlüssels in einer Variante über NFC erfolgen.

In einer weiteren Ausgestaltung des Aspektes kann das MG als Blutzuckermessgerät oder als (quasi-) kontinuierliche Blutzuckermessvorrichtung ausgestaltet sein. Insbesondere bei der OOB-Übertragung von MG zur DV können dieselben beschriebenen Verfahren zum Einsatz gelangen wie oben beschrieben.

In einer weiteren Ausgestaltung des Aspektes kann das System mehrere MGs in Form von Infusions- und Messvorrichtungen umfassen, insbesondere zumindest eine Insulinpumpe und ein Blutzuckermessgerät sowie alternativ oder ergänzend zum Blutzuckermessgeräte eine (quasi-) kontinuierliche Blutzuckermessvorrichtung.

### FIGUREN

In Zusammenhang mit den angehängten Figuren werden nachfolgend bevorzugte Ausführungsformen der Erfindung beschrieben. Diese sollen grundsätzliche Möglichkeiten der Erfindung aufzeigen und keinesfalls einschränkend ausgelegt werden.
- Fig. 1: Symbolische Darstellung von einem Smart Phone und einer Infusionspumpe bei der out-of-band-Schlüsselübertragung von Pumpe auf Smart Phone in einer ersten Ausführungsform. (Phase 1)
- Fig. 2: Symbolische Darstellung der ersten Ausführungsform im Stadium des Aufbaus einer BT Verbindung zwischen Smart Phone und Infusionspumpe nach dem just-works-Prinzip. (Phase 2)
- Fig. 3: Symbolische Darstellung der ersten Ausführungsform beim Übertragen des öffentlichen Schlüssels des Smart Phones über die just-works-BT-Verbindung und des folgenden Aufbaus der End-zu-End-Verschlüsselung in der Applikationsebene. (Phase 3)
- Fig. 4: Sequenzdiagramm für die erste Ausführungsform
- Fig. 5: Sequenzdiagramm für eine zweite Ausführungsform

### FIGURENBESCHREIBUNG

Im Folgenden wird die Erfindung anhand von zwei einfachen Beispielen dargelegt. Dem Fachmann werden dadurch weitere erfindungsgemässe Ausführungen nahegelegt, welche mehr Geräte und Vorrichtungen umfassen. Diese weiteren Ausführungen gehören mit zur Erfindung. Die unten aufgeführten Beispiele der Erfindung sind einfach gehalten, um den grundsätzlichen Erfindungsgedanken klar darlegen zu können.

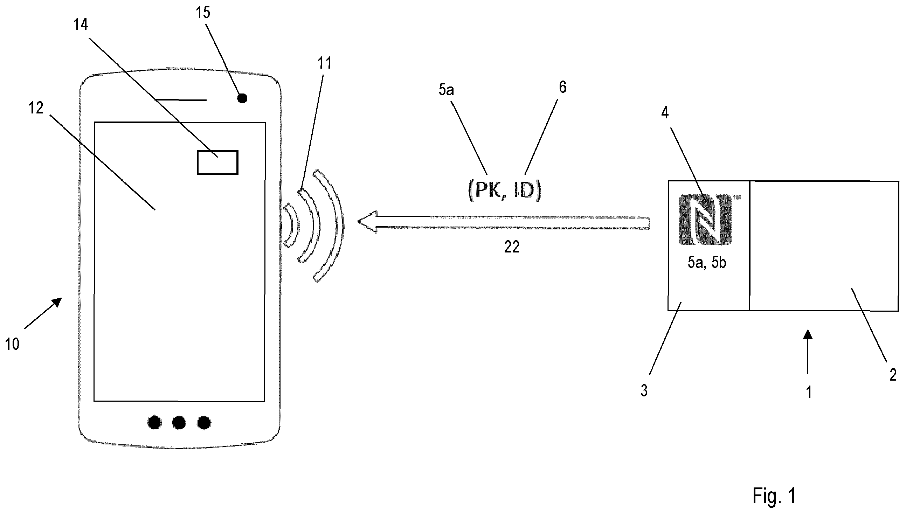

Die Figuren 1 bis 4 beziehen sich auf eine erste Ausführungsform der Erfindung. Bei dieser ersten Ausführungsform umfasst das System zumindest eine als Smart Phone 10 ausgestaltete DV und ein als modulare Patch Pumpe für Insulin 1 ausgestaltetes MG. Die Figuren 1 bis 3 zeigen symbolisch die verschiedenen Phasen der Etablierung der sicheren Daten-Kommunikationsverbindung. Beim Smart Phone 10 kann es sich beispielhaft um ein Galaxy S9 des Herstellers Samsung mit all seinen Eigenschaften und Spezifikationen handeln. Auf eine ausführliche Beschreibung der Eigenschaften und Spezifikationen eines Smart Phones - soweit nicht für die Darlegung der Erfindung notwendig - wird an dieser Stelle verzichtet, da diese öffentlich vorbekannt sind. Die Patch Pumpe 1, begrifflich wird sie im deutschen Sprachraum auch als Pflasterpumpe bezeichnet, ist zum Beispiel in der Patentanmeldung EP 3443996 A1 beschrieben, wobei die EP 3443996 A1 hiermit durch Verweis vollständig in das vorliegende Dokument aufgenommen ist. Die Patch Pumpe ist wie erwähnt modular ausgestaltet und umfasst eine Reservoireinheit 2 und eine Steuereinheit 3, welche lösbar miteinander verbindbar sind. Die Steuereinheit 3 umfasst zumindest einen Teil der Steuerelektronik der Patch Pumpe 1. Insbesondere umfasst die Steuereinheit 3 eine NFC-Einheit 4 für die Nahfeldkommunikation sowie eine Bluetooth-Einheit 7 zum Aufbau und Unterhalt von Bluetooth-Verbindungen mit weiteren Geräten wie zum Beispiel dem Smart Phone 10. Das Smart Phone 10 umfasst insbesondere auch eine NFC-Einheit 11 sowie eine Bluetooth-Einheit 13. Weiter umfasst das Smart Phone 10 auch ein Display 12 sowie zumindest eine Kamera 15. Das Smartphone umfasst auch ein Betriebssystem, z. B. Android, sowie eine App 16, welche dem Datenaustausch mit dem MG und/oder der Steuerung desselben dient. Im hier dargelegten Beispiel der ersten Ausführungsform umfasst die Patch Pumpe 1 kein Display oder Anzeige.

Im Folgenden wird das Verfahren zum Etablieren der erfindungsgemässen sicheren Daten-Kommunikationsverbindung bezugnehmend auf die Figuren 1 bis 4b beschrieben. Das Verfahren durchläuft verschiedene Phasen. Phase 1 ist in Figur 1 dargelegt. In dieser Phase ist die Steuerelektronik der Patch Pumpe aktiviert und hat ein Schlüsselpaar für die zu etablierende End-zu-End-Verschlüsselung dynamisch generiert. Dieses Schlüsselpaar besteht aus einem öffentlichen Schlüssel 5a und einem geheimen Schlüssel 5b. Der öffentliche Schlüssel 5a ist in Phase 1 für externe NFC Lesegeräte lesbar in der NFC Einheit 4 abgelegt, wobei die NFC Einheit 4 dafür ein sogenanntes NFC Tag umfassen kann. Weiter legt die Steuerelektronik der Patch Pumpe 1 ebenfalls Informationen 6 zur Patch Pumpe, wie eine Geräteidentifikation oder eine Seriennummer, ebenfalls lesbar in der NFC-Einheit 4 ab. In Phase 1 wird auf dem Smart Phone die App 16 gestartet und eine darin enthaltene Funktion zur Aufnahme einer Verbindung mit einem MG ausgewählt, wobei NFC-Einheit 11 und Bluetooth-Einheit 13 des Smartphones 10 aktiviert sind. Sobald die App 16 bereit ist, wird das Smart Phone 10 so nahe an die Patch Pumpe 1 heran bewegt, dass die NFC-Einheit 11 des Smart Phones 10 den in der NFC-Einheit 4 abgelegten öffentlichen Schlüssel 5a sowie die Geräteinformationen 6 auslesen und in die App 16 übertragen kann, wobei diese Art der Schlüsselübertrag als out-of-band-Übertragung (OOB) 22 genannt wird, weil die eigentliche Datenkommunikationsverbindung anschliessend über Bluetooth und nicht NFC aufgebaut wird.

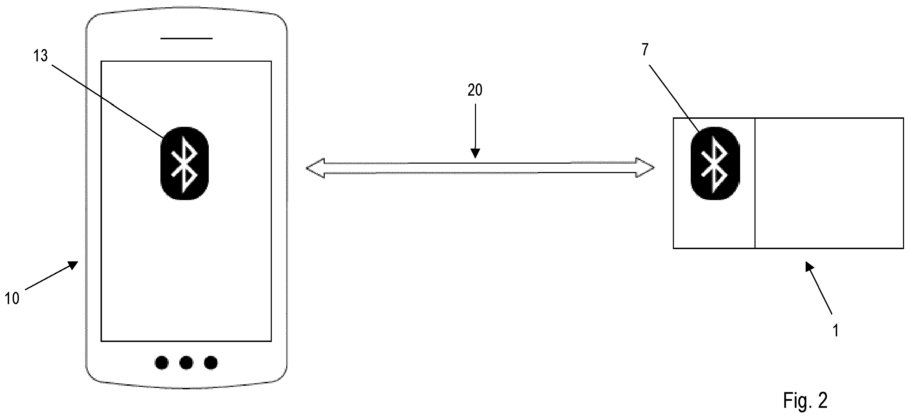

Nach erfolgreicher Übertragung der beschriebenen Daten via NFC veranlasst die App 16 oder die Patch Pumpe 1 in Phase2 (siehe Figur 2) den Aufbau einer Bluetooth-Verbindung 20, insbesondere Bluetooth LE, insbesondere Bluetooth LE Secure Connection, nach dem just-works-Prinzip, wobei zwischen den Bluetooth-Einheiten 7 und 13 eine verschlüsselte Verbindung 20 aufgebaut wird. Beim Aufbau der BT-Verbindung nach dem just-works-Prinzip kommt in vorteilhaften Ausgestaltungen ein Diffie-Hellman-, oder ein Diffie-Hellmann-Merkle-Schlüsseltausch zum Einsatz. Nach Phase 2 besteht also eine verschlüsselte Verbindung zwischen der Patch Pumpe 1 und dem Smart Phone 10. Wichtig dabei ist, dass insbesondere beim Smart Phone 10 die Daten nur bis in die Bluetooth-Einheit 13 verschlüsselt sind, so dass die Möglichkeit besteht, dass auch andere Apps als die App 16 Zugriff auf die Daten haben.

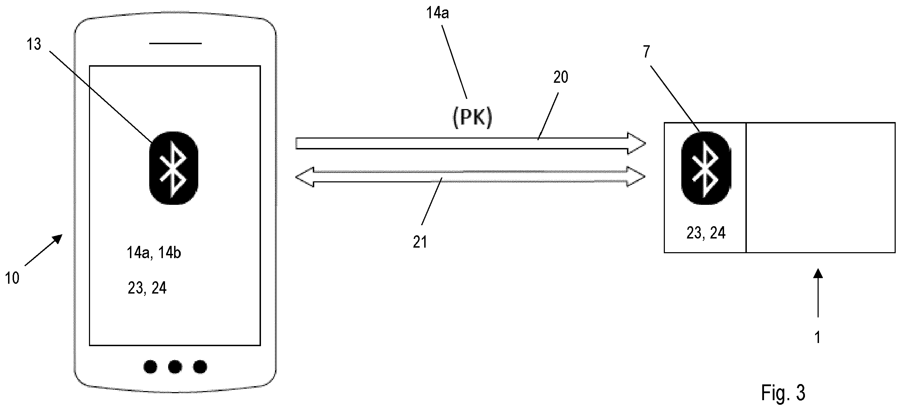

Unter anderem deshalb wird erfindungsgemäss noch eine zusätzliche Verschlüsselung der Daten eingesetzt, welche die Daten erst innerhalb des App-Containers der App 16 entschlüsselt, so dass sichergestellt ist, dass nur die App 16 Zugriff auf die übertragenen Daten hat. Diese zusätzliche Verschlüsselung wird in Phase 3 (siehe Figur 3) etabliert. Hierzu hat auch die App 16 ein Schlüsselpaar generiert, welches aus einem öffentlichen und einem geheimen Schlüssel besteht. Der öffentliche Schlüssel 14a wird nun unverschlüsselt von der App 16 an die Bluetooth-Einheit 13 weitergegeben, welche den Schlüssel 14a über die (verschlüsselte) Bluetooth-Verbindung 20 an die Patch Pumpe 1 sendet, wo die Bluetooth-Einheit 7 den öffentlichen Schlüssel zur weiteren Verarbeitung weitergibt. Nun berechnet die Patch Pumpe 1 aus dem öffentlichen Schlüssel 14a der App 16 (Smart Phone 10) und dem geheimen Schlüssel 5b das gemeinsame Geheimnis 23 nach dem Prinzip des Diffie-Hellman- oder Diffie-Hellman-Merkle-Schlüsseltausch und Geheimnisgenerierung. Aus dem Geheimnis wird schlussendlich ein symmetrischer Langzeitschlüssel 24 abgeleitet, welcher in der App 16 resp. der Patch Pumpe 1 für diese Verbindung hinterlegt (gespeichert) wird. Wie weiter oben erwähnt kann das gemeinsame Geheimnis direkt als Langzeitschlüssel dienen, oder es wird einmalig, wiederholt oder periodisch ein Langzeitschlüssel daraus abgeleitet. Der Langzeitschlüssel 24 dient sodann der End-zu-End-Verschlüsselung zwischen App 16 und Patch Pumpe 1. wobei als Verschlüsselungsverfahren für die End-zu-End-Übertragung der Daten zum Beispiel AES-CCM 128 Bit oder ChaCha20-Poly1305 eingesetzt werden kann.

Typischerweise wird die korrekte Berechnung von Geheimnis und Langzeitschlüssel durch einen Validierungsprozess überprüft. Zum Beispiel kann die App eine Zufallszahl verschlüsselt an die Patch Pumpe senden. Die Patch Pumpe entschlüsselt die Zufallszahl und führt damit eine vorgegebene mathematische Operation aus, und sendet das Resultat verschlüsselt zurück an die App 16. Der App 16 ist die von der Patch Pumpe 1 durchgeführte Operation ebenfalls hinterlegt, so dass das entschlüsselte Resultat in der App 16 überprüft werden kann. Ergänzend kann der Validierungsprozess von der Patch Pumpe 1 aus wiederholt werden, oder er kann grundsätzlich von der Patch Pumpe 1 ausgehen. Nachdem auch die potentielle Validierung erfolgreich abgeschlossen ist, ist die End-zu-End-verschlüsselte Übertragung von Informationen, wie Historiendaten, Einstellungsangaben oder Befehlen (der Begriff Daten ist also breit auszulegen) zwischen App 16 und Patch Pumpe 1 möglich und ein authentischer Austausch End-zu-End-verschlüsselter Daten ist zwischen App und Patch Pumpe möglich. Optional und vorteilhaft können Datenpakete durch das sendende Gerät zusätzlich noch signiert werden.

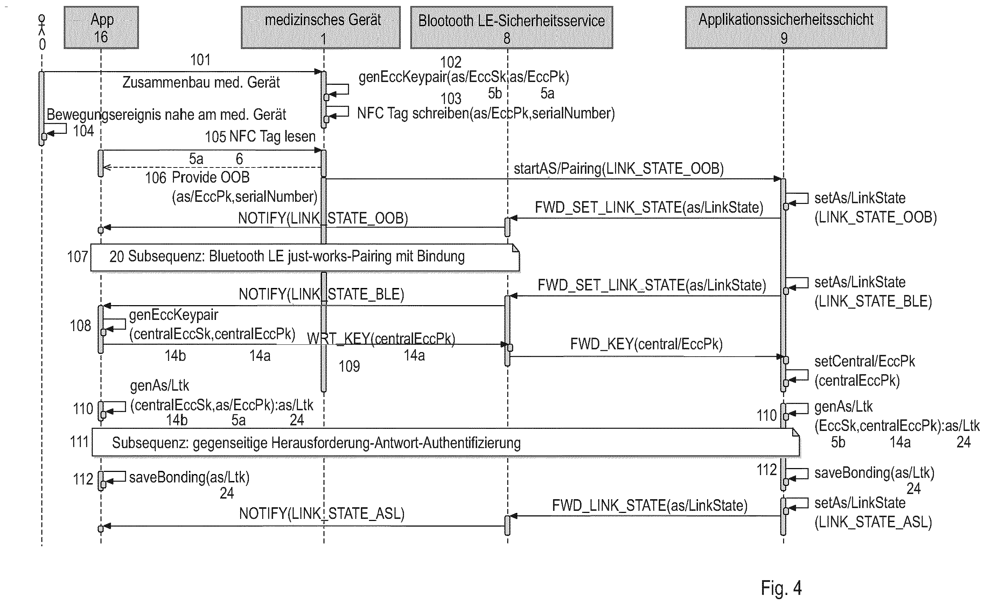

Figur 4 zeigt das dem Verfahren zugrunde liegende Sequenzdiagramm für die Phasen 1, 2 sowie Phase 3 mit Validierung. Das Sequenzdiagramm ist in vier Spalten aufgeteilt: Die benutzende Person 0, die App 16, die Patch Pumpe 1, wobei in Patch Pumpe 1 auch der BT Security Service 8 sowie der Application Security Layer 9 (Applikationsebene) unterschieden werden. Die Ablaufsequenz wird beispielhaft folgend beschrieben, wobei die Beschreibung vereinfacht ist und dem Fachmann auf dem Gebiet die Details aus der Figur 4 offensichtlich sind. Die Sequenz startet, wenn die benutzende Person 0 das medizinische Gerät, hier die Patch Pumpe 1, zusammenbaut (Schritt 101), wodurch die Patch Pumpe aktiviert wird, welche das Schlüsselpaar 5a, 5b generiert (Schritt 102), sowie den generierten öffentlichen Schlüssel 5a sowie Geräteinformation 6 in die NFC-Einheit 4 schreibt (Schritt 103). Die benutzende Person 0 bewegt das Smart Phone 10 nun nahe an die Patch Pumpe 1 heran (Schritt 104), so dass die NFC-Einheit 11 des Smart Phones 10 die NFC-Einheit 4 auslesen kann (Schritt 105), wobei der öffentliche Schlüssel 5a und die Geräteinformation 6 von der Patch Pumpe 1 auf das Smart Phone 10 übertragen werden (Schritt 106). Es folgt nun in Schritt 107 der Aufbau der Bluetooth-just-works-Verbindung 20. Das Smart Phone 10 generiert sodann selber ein Schlüsselpaar 14a, 14b in Schritt 108 und überträgt den öffentlichen Schlüssel 14a über die BT Verbindung 20 auf die Patch Pumpe 1 in Schritt 109. Auf beiden Geräten, der Patch Pumpe 1 und dem Smart Phone 10 (in der App 16), wird nun der Langzeitschlüssel 24 generiert (Schritt 110). Nachdem nun der gemeinsame Langzeitschlüssel 24 bekannt ist, kann über den Challenge-Prozess 111 (auch Challenge-Response-Prozess genannt) eine Validierung der End-zu-End-Verschlüsselung 21 stattfinden. Ist diese Validierung erfolgreich, wird die Sequenz abgeschlossen in dem der Langzeitschlüssel 24 gespeichert wird (Schritt 112).

In einer zweiten Ausführungsform umfasst das System gleich wie bei der ersten Ausführungsform ein Smart Phone 10 als DV. Anders als bei der ersten Ausführungsform ist das MG nun keine Patch Pumpe sondern entweder eine konventionelle Insulinpumpe oder ein Blutzuckermessgerät (beides als 30 bezeichnet). Das MG verfügt in dieser Ausführungsform über eine Anzeige in Form von zum Beispiel eines LCD oder OLED Displays 31. Auf der Anzeige können Text oder graphische Repräsentation (insbesondere als QR-Code oder Barcode) dynamisch dargestellt werden.

Das erfindungsgemässe Verfahren zur Etablierung der sicheren Daten-Kommunikationsverbindung zwischen App 16 und dem MG 30 unterscheidet sich von der ersten Ausführungsform in Phase 1:
Im Unterschied zur ersten Ausführungsform wird der öffentliche Schlüssel 32a des MG 30 nicht via NFC übertragen, sondern bei Bedarf textuell oder als graphische Repräsentation 33 (insbesondere kann die graphische Repräsentation 33 weiter auch Information zum Gerät 37 beinhalten) auf der Anzeige angezeigt. Die Übertragung erfolgt optisch, indem die zumindest eine am Smart Phone 10 angeordnete Kamera 15 die Anzeige 31 des MG 30 scannt und die App 16 aus dem gescannten Bild die Repräsentation 33 des öffentlichen Schlüssels 32a sowie Geräteinformation 37 extrahiert und folgend den Schlüssel 32a an sich generiert. Der Schlüssel 32a wird dann analog zur ersten Ausführungsform weiterverwendet. Phasen 2 und 3 sind bei der zweiten Ausführungsform gleich wie bei der ersten Ausführungsform. Es wird entsprechend darauf verwiesen.

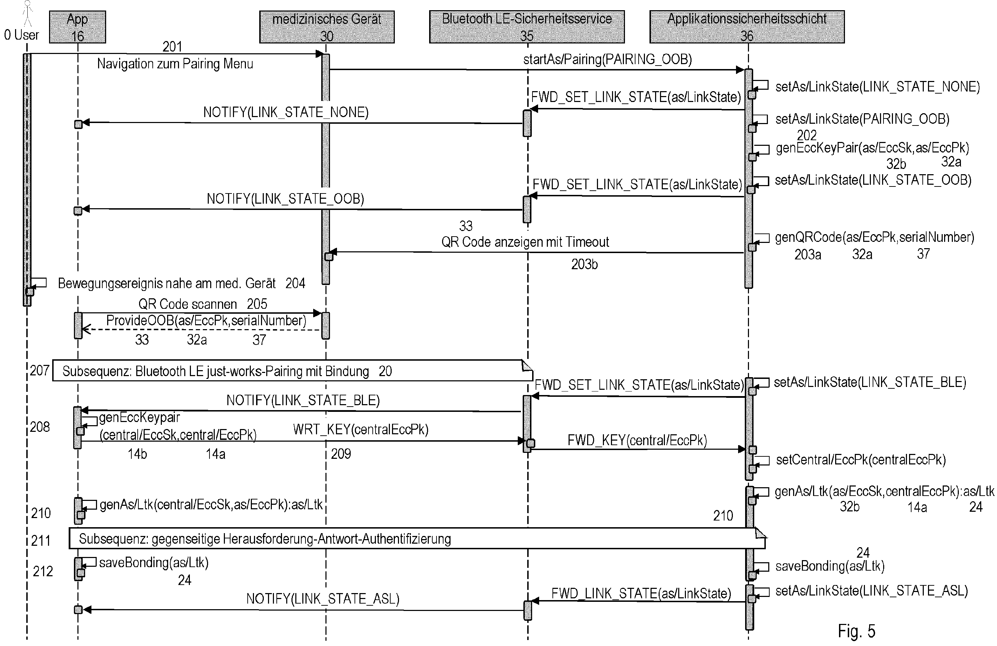

Figur 5 zeigt das entsprechende Sequenzdiagramm für die zweite Ausführungsform. Die benutzende Person 0 navigiert in Schritt 201 im Menu des MG 30 in das Pairing Menu, um den Pairingvorgang zu starten. Anschliessend wird in Schritt 202 das Schlüsselpaar 32a, 32b generiert. Weiter generiert das MG im nächsten Schritt 203a die graphische Repräsentation 33 (hier beispielhaft einen QR code) aus dem öffentlichen Schlüssels 32a und zugehöriger Geräteinformation 37, welche danach auf der Anzeige 31 dargestellt wird (Schritt 203b). Die benutzende Person 0 bewegt das Smart Phone 10 mit Kamera 15 zum MG 30 hin (Schritt 204) und scannt sodann mit der Kamera 15 die graphische Repräsentation 33 (Schritt 205). Die weitere Sequenz folgt analog der ersten Ausführungsform. Entsprechend wird in Schritt 207 die BT-just-works-Verbindung 20 aufgebaut. Folgend wird im Smartphone 10 das Schlüsselpaar 14a, 14b generiert (Schritt 208) und danach der öffentliche Schlüssel 14a auf das MG 30 via die BT-Verbindung 20 übertragen (Schritt 209). MG und Smart Phone generieren folgend den Langzeitschlüssel 24 (Schritt 210) und validieren diesen über den Challenge-Prozess 211. Schlussendlich und nach erfolgreicher Validierung der End-zu-End-Verschlüsselung wird der Langzeitschlüssel 24 in Schritt 212 in beiden Geräten 10 und 30 gespeichert.

### BEZUGSZEICHENLISTE

- 0: Benutzende Person

- 1: Patch Pumpe
- 2: Reservoireinheit
- 3: Steuereinheit
- 4: NFC-Einheit

- 5a: öffentlicher Schlüssel
- 5b: geheimer Schlüssel
- 6: Geräteinformationen
- 7: Bluetooth-Einheit
- 8: Bluetooth Security Service
- 9: Application Security Layer (MG)

- 10: Smart Phone
- 11: NFC-Einheit
- 12: Display
- 13: Bluetooth-Einheit
- 14a: öffentlicher Schlüssel
- 14b: geheimer Schlüssel
- 15: Kamera
- 16: App

- 20: Bluetooth-just-works-Verbindung
- 21: End-zu-End-verschlüsselte-Verbindung über Bluetooth
- 22: Ouf-of-band-Übertragung
- 23: gemeinsames Geheimnis
- 24: Langzeitschlüssel

- 30: MG (Insulinpumpe oder Blutzuckermessgerät)
- 31: Anzeige
- 32a: öffentlicher Schlüssel
- 32b: geheimer Schlüssel
- 33: graphische Repräsentation von 32a
- 34: Bluetooth Security Layer
- 36: Application Security Layer
- 37: Geräteinformationen

- 101: Zusammenbau Patch Pumpe 1
- 102: Generieren von öffentlichem 5a und geheimem 5b Schlüssel in Patch Pumpe 1
- 103: Beschreiben NFC-Einheit 4 mit öffentlichem Schlüssel 5a und Geräteinformation 6
- 104: Bewegen von Smart Phone 10 in Nähe von Patch Pumpe 1
- 105: Lesen von NFC-Einheit 4 durch NFC-Einheit 11
- 106: Übertragung von öffentlichem Schlüssel 5a und Geräte Information 6
- 107: Aufbau Bluetooth-just-works-Verbindung
- 108: Generieren von öffentlichem 14a und geheimem 14 Schlüssel in Smart Phone 10
- 109: Übertragung von öffentlichem Schlüssel 14a via Bluetooth Verb. 20
- 110: Generieren von Langzeitschlüssel 24
- 111: Challenge-Prozess
- 112: Speichern des Langzeitschlüssels 24

- 201: Navigieren zum Pairing Menu
- 202: Generieren von öffentlichem 32a und geheimem 32b Schlüssel in MG 30
- 203a: Generieren der graphischen Repräsentation 33
- 203b: Anzeigen der graphischen Repräsentation 33 auf Display 31
- 204: Bewegen von Smart Phone 10 vor das MG 30
- 205: Scannen des Displays 31 mit Kamera 15
- 207: Aufbau Bluetooth-just-works-Verbindung
- 208: Generieren von öffentlichem 14a und geheimem 14 Schlüssel in Smart Phone 10
- 209: Übertragung von öffentlichem Schlüssel 14a via Bluetooth Verb. 20
- 210: Generieren von Langzeitschlüssel 24
- 211: Challenge-Prozess
- 212: Speichern des Langzeitschlüssels 24

## Patentansprüche

1. Verfahren zum Etablieren einer sicheren Daten-Kommunikationsverbindung zwischen einem portablen medizinischen Gerät, insbesondere einem Infusionsgerät, einem Injektionsgerät oder einer Blutzuckermessvorrichtung, und einer Datenmanagementvorrichtung, wobei die Datenmanagementvorrichtung und das medizinische Gerät jeweils eine Bluetooth-Einheit umfassen, **dadurch gekennzeichnet, dass** das Verfahren zumindest folgende Schritte umfasst:
• out-of-band-Übertragung eines öffentlichen Schlüssels eines Schlüsselpaars des medizinischen Geräts und von Geräteinformation vom medizinischen Gerät auf die Datenmanagementvorrichtung, wobei die Übertragung also nicht über Bluetooth erfolgt,
• Aufbau einer verschlüsselten Bluetooth -Daten-Kommunikationsverbindung nach dem just-works-Prinzip (just-works-Verbindung) zwischen dem medizinischen Gerät und der Datenmanagementvorrichtung,
• Übertragung eines öffentlichen Schlüssels eines Schlüsselpaars der Datenmanagementvorrichtung von der Datenmanagementvorrichtung auf das medizinische Gerät über die aufgebaute und verschlüsselte Bluetooth-Verbindung,
• Berechnen eines kombinierten Schlüssels auf der Datenmanagementvorrichtung aus dem übertragenen öffentlichen Schlüssel des medizinischen Geräts und eines geheimen Schlüssels des Schlüsselpaars der Datenmanagementvorrichtung,
• Berechnen desselben kombinierten Schlüssels auf dem medizinischen Gerät aus dem übertragenen öffentlichen Schlüssel der Datenmanagementvorrichtung und eines geheimen Schlüssels des Schlüsselpaares des medizinischen Geräts und
• Aufbau einer End-zu-End-verschlüsselten Verbindung zwischen dem medizinischen Gerät und der Datenmanagementvorrichtung unter Nutzung des kombinierten, vorzugsweise symmetrischen Schlüssels, wobei so End-zu-End-verschlüsselte Daten über die verschlüsselte Bluetooth-Verbindung übertragen werden.

2. Verfahren nach Anspruch 1, wobei die out-of-band-Übertragung des öffentlichen Schlüssels mittels Nahfeldkommunikation, insbesondere NFC, erfolgt.

3. Verfahren nach Anspruch 1, wobei die out-of-band-Übertragung des öffentlichen Schlüssels mittels Kamera der Datenmanagementvorrichtung erfolgt, welche den Schlüssel, welcher vom medizinischen Gerät angezeigt wird, optisch erfasst.

4. Verfahren nach Anspruch 1, wobei die out-of-band-Übertragung des öffentlichen Schlüssels mittels Kamera der Datenmanagementvorrichtung erfolgt, welche den Schlüssel, welcher am medizinischen Gerät oder auf dessen Oberfläche, insbesondere dauerhaft, angeordnet ist, optisch erfasst.

5. Verfahren nach den Ansprüchen 1 bis 3, wobei öffentlicher und geheimer Schlüssel von mindestens einem aus medizinischem Gerät und Datenmanagementvorrichtung als Schlüsselpaar dynamisch generierbar sind.

6. Verfahren nach einem der vorhergehenden Ansprüche, wobei der Aufbau der Bluetooth-Verbindung nach dem just-works-Prinzip funktioniert und für die Verschlüsselung ein Diffie-Hellman-, oder ein Diffie-Hellmann-Merkle-Schlüsseltausch stattfindet.

7. Verfahren nach Anspruch 6, wobei die Bluetooth-Verbindung eine Bluetooth LE-Verbindung ist, insbesondere eine mit Bluetooth LE Secure Connection just-works aufgebaute Verbindung, und zum Schlüsselaustausch vorzugsweise beim Aufbau der Bluetooth LE Secure Connection just-works-Verbindung Elliptic-Curve Diffie-Hellman (ECDH) P-256 eingesetzt wird, wobei aus dem durch ECDH berechneten gemeinsamen Schlüssel ein dauerhafter Schlüssel mit 128 Bit Länge bestimmt wird.

8. Verfahren nach einem der vorhergehenden Ansprüche, wobei die End-zu-End-verschlüsselte Verbindung nach erfolgtem Aufbau validiert wird und der kombinierte Schlüssel folgend in medizinischem Gerät und Datenmanagementvorrichtung gespeichert wird.

9. Verfahren nach Anspruch 2, wobei das medizinische Gerät durch ein Nahfeldkommunikationssignal der Datenmanagementvorrichtung aktiviert wird und von einem Energiesparmodus oder Standby-Modus in einen Betriebsmodus versetzt wird.

10. Verfahren nach Anspruch 9, wobei im medizinischen Gerät das Schlüsselpaar des medizinischen Geräts aus einem öffentlichen Schlüssel und einem geheimen Schlüssel nach dem Wechsel in den Betriebsmodus dynamisch generierbar ist, und dieses Schlüsselpaar für den Aufbau der End-zu-End-verschlüsselten Verbindung verwendbar ist.

11. Verfahren nach Anspruch 3, wobei das medizinische Gerät über ein Display verfügt, auf welchem der öffentliche Schlüssel des medizinischen Geräts sowie die Geräteinformation in Form einer graphischen Repräsentation anzeigbar ist, so dass er mit der Kamera der Datenmanagementvorrichtung erfassbar ist, wobei die graphische Repräsentation insbesondere ein Barcode, ein QR-Code oder eine Anordnung von alphanumerischen Zeichen sein kann.

12. Verfahren nach Anspruch 11, wobei das medizinische Gerät Bedienelemente umfasst, mit welchen eine benutzende Person die Anzeige der graphischen Repräsentation aktiv forcieren kann.

13. Verfahren Anspruch 11 oder 12, wobei der öffentliche Schlüssel und die graphische Repräsentation dynamisch generierbar sind.

14. System bestehend aus mindestens einem portablen medizinischen Gerät , insbesondere einem Infusionsgerät, einem Injektionsgerät und/oder einer Blutzuckermessvorrichtung, und einer Datenmanagementvorrichtung , wobei die Datenmanagementvorrichtung ein Mobiltelefon oder Smart Phone ist, auf welchem eine App installiert ist, in welcher Messwerte Therapiedaten und/oder Therapieparameter gespeichert, eingetragen und/oder bearbeitet werden können,
• wobei Daten über eine drahtlose Bluetooth-Verbindung zwischen der Datenmanagementvorrichtung und dem mindestens einen medizinischen Gerät austauschbar sind,
• **dadurch gekennzeichnet, dass** die Bluetooth-Verbindung mit zusätzlicher End-zu-End-Verschlüsselung nach einem Verfahren nach einem der vorhergehenden Ansprüche sicher etabliert ist.

15. System nach dem vorhergehenden Anspruch, wobei das System aus einem Smart Phone, einer Insulininjektionsvorrichtung oder einer Insulininfusionsvorrichtung sowie einem Blutzuckermessgerät besteht, und wobei vom Smart Phone zu jedem der weiteren Geräte eine verschlüsselte Verbindung nach einem Verfahren der Ansprüche 1 bis 8 etabliert ist.

16. System nach dem vorhergehenden Anspruch, wobei das System weiter eine kontinuierliche oder quasi-kontinuierliche Blutzuckermessvorrichtung umfasst.

17. System nach Anspruch 15 oder 16, wobei vom Smart Phone zu den zusätzlichen Geräten des Systems Verbindungen nach jeweils unterschiedlichen Verfahren der Ansprüche 1 bis 8 etabliert sind.

## Claims

1. Method for establishing a secure data communication connection between a portable medical device, in particular an infusion device, an injection device or a blood glucose measuring apparatus, and a data management apparatus, the data management apparatus and the medical device each comprising a Bluetooth unit, **characterized in that** the method comprises at least the following steps:
• out-of-band transmission of a public key of a key pair of the medical device and device information from the medical device to the data management apparatus, the transmission therefore not taking place via Bluetooth,
• establishing an encrypted Bluetooth data communication connection according to the just-works principle (just-works connection) between the medical device and the data management apparatus,
• transmitting a public key of a key pair of the data management apparatus from the data management apparatus to the medical device via the established and encrypted Bluetooth connection,
• calculating a combined key on the data management apparatus from the transmitted public key of the medical device and a secret key of the key pair of the data management apparatus,
• calculating the same combined key on the medical device from the transmitted public key of the data management apparatus and a secret key of the key pair of the medical device and
• establishing an end-to-end encrypted connection between the medical device and the data management apparatus using the combined, preferably symmetric key, end-to-end encrypted data being transmitted via the encrypted Bluetooth connection.

2. Method according to claim 1, wherein the out-of-band transmission of the public key is carried out by means of near-field communication, in particular NFC.

3. Method according to claim 1, wherein the out-of-band transmission of the public key is carried out by means of a camera of the data management apparatus, which optically captures the key displayed by the medical device.

4. Method according to claim 1, wherein the out-of-band transmission of the public key is carried out by means of a camera of the data management apparatus, which optically captures the key arranged, in particular permanently, on the medical device or on its surface.

5. Method according to claims 1 to 3, wherein public and secret keys of at least one of the medical device and the data management apparatus can be dynamically generated as a key pair.

6. Method according to any of the preceding claims, wherein the establishment of the Bluetooth connection functions according to the just-works principle and a Diffie-Hellman or a Diffie-Hellman-Merkle key exchange takes place for the encryption.

7. Method according to claim 6, wherein the Bluetooth connection is a Bluetooth **LE** connection, in particular a connection established with Bluetooth LE Secure Connection just-works, and Elliptic-Curve Diffie-Hellman (ECDH) P-256 is used for key exchange, preferably when establishing the Bluetooth **LE** Secure Connection just-works connection, wherein a permanent key having a length of 128 bits is determined from the common key calculated by ECDH.

8. Method according to any of the preceding claims, wherein the end-to-end encrypted connection is validated after it has been established and the combined key is subsequently stored in the medical device and the data management apparatus.

9. Method according to claim 2, wherein the medical device is activated by a near-field communication signal of the data management apparatus and is switched from a power saving mode or standby mode to an operating mode.

10. Method according to claim 9, wherein in the medical device the key pair of the medical device can be dynamically generated from a public key and a secret key after the change to the operating mode, and this key pair can be used for establishing the end-to-end encrypted connection.

11. Method according to claim 3, wherein the medical device has a display on which the public key of the medical device and the device information can be displayed in the form of a graphical representation so that it can be captured by the camera of the data management apparatus, wherein the graphical representation can in particular be a barcode, a QR code or an arrangement of alphanumeric characters.

12. Method according to claim 11, wherein the medical device comprises operating elements with which a user can actively force the display of the graphical representation.

13. Method claim 11 or 12, wherein the public key and the graphical representation can be dynamically generated.

14. System consisting of at least one portable medical device, in particular an infusion device, an injection device and/or a blood glucose measuring apparatus, and a data management apparatus, the data management apparatus being a mobile phone or smart phone on which an app is installed, in which measured values, therapy data and/or therapy parameters can be stored, entered and/or edited,
• data being exchangeable between the data management apparatus and the at least one medical device via a wireless Bluetooth connection,
• **characterized in that** the Bluetooth connection is securely established with additional end-to-end encryption in accordance with a method according to any of the preceding claims.

15. System according to the preceding claim, wherein the system consists of a smartphone, an insulin injection apparatus or an insulin infusion apparatus and a blood glucose meter, and wherein an encrypted connection is established from the smartphone to each of the further devices according to a method of claims 1 to 8.

16. System according to the preceding claim, wherein the system further comprises a continuous or quasi-continuous blood glucose measuring apparatus.

17. System according to claim 15 or 16, wherein connections are established from the smartphone to the additional devices of the system according to different methods of claims 1 to 8.

## Revendications

1. Procédé permettant d'établir une connexion de communication de données sécurisée entre un appareil médical portable, en particulier un appareil de perfusion, un appareil d'injection ou un dispositif de mesure de glycémie, et un dispositif de gestion de données, dans lequel le dispositif de gestion de données et l'appareil médical comprennent respectivement une unité Bluetooth, **caractérisé en ce que** le procédé comprend au moins les étapes suivantes :
• transmission hors bande d'une clé publique d'une paire de clés de l'appareil médical et d'informations d'appareil, par l'appareil médical au dispositif de gestion de données, dans lequel la transmission n'est donc pas effectuée par l'intermédiaire de Bluetooth,
• établissement d'une connexion de communication de données Bluetooth chiffrée selon le principe just works (connexion just works) entre l'appareil médical et le dispositif de gestion de données,
• transmission d'une clé publique d'une paire de clés du dispositif de gestion de données, par le dispositif de gestion de données à l'appareil médical, par l'intermédiaire de la connexion Bluetooth établie et chiffrée,
• calcul d'une clé combinée sur le dispositif de gestion de données à partir de la clé publique transmise de l'appareil médical et d'une clé secrète de la paire de clés du dispositif de gestion de données,
• calcul de la même clé combinée sur l'appareil médical à partir de la clé publique transmise du dispositif de gestion de données et d'une clé secrète de la paire de clés de l'appareil médical, et
• établissement d'une connexion chiffrée de bout en bout entre l'appareil médical et le dispositif de gestion de données en utilisant la clé combinée, de préférence symétrique, dans lequel les données chiffrées de bout en bout sont ainsi transmises par l'intermédiaire de la connexion Bluetooth chiffrée.

2. Procédé selon la revendication 1, dans lequel la transmission hors bande de la clé publique est effectuée au moyen d'une communication en champ proche, en particulier CCP.

3. Procédé selon la revendication 1, dans lequel la transmission hors bande de la clé publique est effectuée au moyen d'une caméra du dispositif de gestion de données, laquelle détecte de manière optique la clé affichée par l'appareil médical.

4. Procédé selon la revendication 1, dans lequel la transmission hors bande de la clé publique est effectuée au moyen d'une caméra du dispositif de gestion de données, laquelle détecte de manière optique la clé qui est disposée, en particulier de manière permanente, sur l'appareil médical ou sur sa surface.

5. Procédé selon les revendications 1 à 3, dans lequel la clé publique et la clé secrète d'au moins l'un parmi un appareil médical et un dispositif de gestion de données peuvent être générées de manière dynamique en tant que paire de clés.

6. Procédé selon l'une des revendications précédentes, dans lequel l'établissement de la connexion Bluetooth fonctionne selon le principe just works et un échange de clés Diffie-Hellman, ou Diffie-Hellman-Merkle, a lieu pour le chiffrage.

7. Procédé selon la revendication 6, dans lequel la connexion Bluetooth est une connexion Bluetooth LE, en particulier une connexion établie avec Bluetooth LE Secure Connection just works, et pour l'échange de clés, un Diffie-Hellman à courbes elliptiques (ECDH) P-256 est utilisé, de préférence lors de l'établissement de la connexion Bluetooth LE Secure Connection just works, dans lequel une clé permanente de longueur 128 bits est déterminée à partir de la clé commune calculée par ECDH.

8. Procédé selon l'une des revendications précédentes, dans lequel la connexion chiffrée de bout en bout est validée après un établissement réussi et la clé combinée est ensuite enregistrée dans l'appareil médical et le dispositif de gestion de données.

9. Procédé selon la revendication 2, dans lequel l'appareil médical est activé par un signal de communication en champ proche du dispositif de gestion de données et passe d'un mode économie d'énergie ou d'un mode veille à un mode de fonctionnement.

10. Procédé selon la revendication 9, dans lequel, dans l'appareil médical, la paire de clés de l'appareil médical constituée d'une clé publique et d'une clé secrète peut être générée de manière dynamique après le passage au mode de fonctionnement, et ladite paire de clés peut être utilisée pour l'établissement de la connexion chiffrée de bout en bout.

11. Procédé selon la revendication 3, dans lequel l'appareil médical possède un écran sur lequel la clé publique de l'appareil médical ainsi que les informations d'appareil peuvent être affichées sous la forme d'une représentation graphique de sorte qu'il peut être détecté par la caméra du dispositif de gestion de données, dans lequel la représentation graphique peut en particulier être un code à barres, un code QR ou un agencement de caractères alphanumériques.

12. Procédé selon la revendication 11, dans lequel l'appareil médical comprend des éléments de manipulation avec lesquels une personne utilisatrice peut forcer activement l'affichage de la représentation graphique.

13. Procédé revendication 11 ou 12, dans lequel la clé publique et la représentation graphique peuvent être générées de manière dynamique.

14. Système constitué d'au moins un appareil médical portable, en particulier un appareil de perfusion, un appareil d'injection et/ou un dispositif de mesure de glycémie, et d'un dispositif de gestion de données, dans lequel le dispositif de gestion de données est un téléphone mobile ou un mobile multifonction sur lequel est installée une application dans laquelle des valeurs de mesure, des données de thérapie et/ou des paramètres de thérapie peuvent être mémorisés, inscrits et/ou traités,
• dans lequel des données peuvent être échangées par l'intermédiaire d'une connexion Bluetooth sans fil entre le dispositif de gestion de données et l'au moins un appareil médical,
• **caractérisé en ce que** la connexion Bluetooth est établie de manière sécurisée avec un chiffrage de bout en bout supplémentaire selon un procédé selon l'une des revendications précédentes.

15. Système selon la revendication précédente, dans lequel le système est constitué d'un mobile multifonction, d'un dispositif d'injection d'insuline ou d'un dispositif de perfusion d'insuline et d'un appareil de mesure de glycémie, et dans lequel une connexion chiffrée est établie entre le mobile multifonction et chacun des autres appareils selon un procédé selon les revendications 1 à 8.

16. Système selon la revendication précédente, dans lequel le système comprend en outre un dispositif de mesure de glycémie continu ou quasi-continu.

17. Système selon la revendication 15 ou 16, dans lequel des connexions sont établies entre le mobile multifonction et les appareils supplémentaires du système selon des procédés respectivement différents selon les revendications 1 à 8.
